# FASCICULE DE BREVET EUROPEEN

(11) **EP 0 837 047 B1**
(45) Date de publication et mention de la délivrance du brevet: **12.12.2001**
(21) Numéro de dépôt: 97402422.6
(22) Date de dépôt: 14.10.1997
(51) Int. Cl.: C07C 17/389, C07C 19/08

(54) **Purification du pentafluoroéthane**
Reinigung von Pentafluorethan
Purification of pentafluoroethane

(30) Priorité: 18.10.1996 FR 9612714
(43) Date de publication de la demande: 22.04.1998
(73) Titulaire: Atofina, 92800 Puteaux (FR)
(72) Inventeur: Bertocchio, René, 69390 Vourles Par Vernaison (FR); Lantz, André, 69390 Vernaison (FR)
(74) Mandataire: Leboulenger, Jean

(56) Documents cités:
- EP-A- 0 389 334
- WO-A-94/22793

## Description

La présente invention concerne le domaine des hydrofluoroalcanes (HFA) et a plus particulièrement pour objet la purification du pentafluoroéthane contenant du chlorotrifluoroéthylène.

Le pentafluoroéthane (connu dans le métier sous la désignation F125) a été proposé comme substitut du chloropentafluoroéthane (F115) et du chlorodifluorométhane (F22) pour les applications liées aux industries du froid ; seul ou en association avec d'autres HFA, il est notamment intéressant pour remplacer le réfrigérant R502 (mélange azéotropique de F115 et de F22) très utilisé jusqu'à présent pour la production de froid industriel.

Le F125 peut être obtenu par hydrogénolyse du F115 comme cela est décrit dans le brevet EP 506 525 ou par fluoruration en phase liquide ou gazeuse de composés chlorés ou chlorofluorés. Parmi ces procédés, on peut citer plus particulièrement ceux partant d'oléfines comme le perchloroéthylène avec addition d'une première molécule d'HF et substitution des autres atomes de chlore comme décrit dans le brevet US 3 755 477 ; ainsi qu'il est indiqué dans le brevet EP 456 552 on peut partir également de produits de fluoruration intermédiaires tels que le 1,1,1-trifluoro-dichloroéthane (F123) et le 1,1,1,2-tétrafluoro-chloroéthane (F124).

Ces procédés, en particulier ceux mettant en oeuvre des températures élevées, génèrent des oléfines bien connues pour leur toxicité et également pour leur réactivité. Même à des concentrations relativement faibles, ces oléfines peuvent par exemple s'oxyder au contact de l'air et conduire à des produits de transformation acides rendant le F125 impropre à sa commercialisation.

La demande de brevet EP 389334 décrit la purification du 1,1,1,2-tetrafluoroethane (R-134a) par élimination du 2-chlore-1,1-difluoroéthylène (R-1122) par mise en contact d'un courant de R-134a avec un charbon actif. Ce document ne donne cependant aucune précision sur le type de charbon actif à utiliser.

Après distillation, le pentafluoroéthane peut encore renfermer du chlorotrifluoroéthylène (F1113) provenant de la déchlorhydratation du 1,2-dichloro-1,1,2-trifluoroéthane (F123a), un sous-produit normal de la fluoruration du perchloroéthylène, ou de la deshydrofluoration des chloro-tétrafluoroéthanes (F124 et F124a). Durant un stockage prolongé du F125 et en présence des traces d'air normalement dissoutes dans le F125 liquide, le F1113 peut aisément s'oxyder et, au contact de l'humidité résiduelle, conduire à la formation de produits acides tels que HCI, HF et CF₃COOH. D'autre part, outre sa sensibilité à l'oxydation, le F1113 est bien connu pour son aptitude à la polymérisation ou à la copolymérisation en présence d'autres oléfines.

Il a maintenant été trouvé que le F1113 résiduel dans un F125 peut être éliminé par passage du F125, en phase gazeuse ou liquide, à travers un lit d'un charbon actif spécifique et que le F1113 peut ensuite être désorbé sans perte d'efficacité et de capacité de l'adsorbant.

L'invention a donc pour objet un procédé de purification d'un F125 contenant du F1113, caractérisé en ce que l'on fait passer un courant du F125 à purifier à travers un lit de charbon actif ayant une surface spécifique comprise entre 800 et 150 m²/g et une microporosité de l'ordre de 50 à 90%, de préférence 70 à 85%.

Le pourcentage de microporosité est par définition la fraction de surface correspondant aux pores de diamètre inférieur ou égal à 2 nm. Ces charbons actifs peuvent être utilisés tels quels après séchage à 135-145°C et, après utilisation, ils peuvent être régénérés sous pression réduite à une température d'au moins 200°C ou par balayage sous un courant de gaz inerte tel que l'azote ou l'hélium, jusqu'à une température finale d'environ 250°C. Dans ce cas, la mise en température est de préférence faite progressivement et par paliers à partir de 100°C afin d'éviter toute transformation du F1113 sur l'adsorbant.

Le procédé selon l'invention peut s'appliquer à la purification d'un F125 contenant jusqu'à 10000 ppm de F1113, de préférence de 10 à 1000 ppm, ainsi que des quantités variables d'impuretés saturées comme le F124.

Le traitement selon l'invention peut être effectué en phase gazeuse ou en phase liquide à une température comprise entre -20 et +80°C, de préférence entre 10 et 40°C, et sous une pression allant de 100 à 2200 kPa.

Pour le traitement en phase gazeuse, on utilise un débit correspondant à une vitesse spatiale comprise entre 50 et 1500 v/h/v (volume de F125/heure/volume apparent d'adsorbant) avec une vitesse de passage de 10 à 2500 cm/min. En phase liquide, ces vitesses sont réduites respectivement à 4-20 v/h/v et 5-40 cm/minute.

Les exemples suivants illustrent l'invention sans la limiter. Les ppm indiquées sont exprimées en poids.

### EXEMPLE 1

Dans un tube en acier inoxydable de 50 cm de hauteur et 30 mm de diamètre intérieur comportant au tiers de sa hauteur une grille métallique, on a placé une charge de 36 g de charbon activé CECA AC 35 en granulés de 3 mm, puis on y a fait passer, à température ambiante et au débit de 10 l/h, un courant gazeux de F125 brut renfermant 17 ppm de F1113 et 970 ppm de F115.

Après deux heures de fonctionnement, on ne détectait aucune trace de F1113 (<1 ppm) en sortie de l'épurateur et seulement 350 ppm de F115.

Au bout de 4 heures, l'élimination du F1113 était toujours quantitative, mais la concentration en F115 était revenue à sa valeur initiale.

Le charbon CECA AC 35 a une densité apparente de 0,45 g/ml, une surface spécifique de 1335 m²/g et une microporosité de 78 %.

### EXEMPLE 2

Dans le même appareillage que celui utilisé à l'exemple 1, on a placé une charge de 34 g de charbon activé NORIT RDBX 1.5 sur laquelle on a fait passer ensuite, à température ambiante et au débit de 4 l/h, un courant gazeux d'un F125 brut renfermant 245 ppm de F1113 et 2,4 % de F124.

Le point de rupture pour le F1113 est apparu dans ces conditions au bout de 31 heures de marche, ce qui correspond à une capacité de 0,5 g de F1113 retenu pour 100 g de charbon sec.

Le Norit RDBX 1.5 a une densité apparente de 0,443 g/ml et une surface spécifique de 1358 m²/g pour un taux de microporosité (diamètre de pore ≤ 2 nm) de 85 %.

### EXEMPLE 3

Dans un tube en acier inoxydable de 50 cm de hauteur et 30 mm de diamètre interne on a placé une charge de 33,4 g (75 ml) de charbon actif CECA GAC 1240 Plus, puis on y a fait passer à température ambiante et au débit de 4 l/h un courant gazeux de F125 brut renfermant 305 ppm de F1113, 1050 ppm de F115, 71 ppm de F124, 24 ppm de F23 et 39 ppm de F143a.

Après passage sur le lit de charbon, le F1113 et le F124 étaient entièrement éliminés. Le F124 n'est réapparu qu'après 16 heures de marche et le F1113 au bout de 41 heures (concentration résiduelle > 1 ppm). A ce point, la capacité de rétention en F1113 du charbon s'élevait à 0,74 %.

La charge de charbon a alors été retirée de l'épurateur et chauffée durant 2 heures à 200°C sous pression réduite (0,1 kPa) puis remise en place pour y faire passer à nouveau un même courant de F125 brut. Lors de ce nouveau cycle d'adsorption, on a observé la même élimination totale du F124 et du F1113, le point de rupture pour ce dernier n'apparaissant qu'au bout de 37 heures, ce qui correspond à une capacité d'adsorption de 0,73 %.

Cet exemple montre que le charbon actif est parfaitement régénérable après un cycle d'adsorption et qu'il conserve toute son efficacité et sa capacité.

Le charbon GAC 1240 (CECA) a une densité apparente de 0,443 g/ml, une surface spécifique de 1284 m²/g et un taux de microporosité (pores de diamètre ≤ 2 nm) de 72 %.

### EXEMPLE 4

On a fait passer, au débit de 17,3 l/h, 432 g de F125 brut renfermant 300 ppm de F1113, au travers d'un lit de charbon actif GAC 1240 Plus en grains de 0,5-1,5 mm ; le tube épurateur d'un diamètre interne de 8 mm et d'une hauteur utile de 35 cm renfermait 7,6 g de charbon actif.

Un échantillon du F125 traité, prélevé en sortie de l'épurateur, au bout de 100 minutes de fonctionnement, ne renfermait aucune trace détectable de F1113 (< 1 ppm), ce qui confirme l'efficacité du procédé aux régimes de marche élevés.

## Revendications

1. Procédé de purification d'un pentafluoroéthane (F125) contenant du chlorotrifluoroéthylène (F1113) **caractérisé en ce que** l'on fait passer un courant du F125 à purifier à travers un lit de charbon actif, ledit charbon actif ayant une surface spécifique comprise entre 800 et 1500 m²/g et une microporosité de l'ordre de 50 à 90 %, de préférence 70 à 85 %.

2. Procédé selon la revendication 1 dans lequel on opère à une température comprise entre -20 et +80°C, de préférence entre 10 et 40°C, et sous une pression allant de 100 à 2200 kPa.

3. Procédé selon la revendication 2 dans lequel on opère en phase gazeuse avec un débit de F125 à purifier correspondant à une vitesse spatiale comprise entre 50 et 1500 v/h/v, avec une vitesse de passage de 10 à 2500 cm/min.

4. Procédé selon la revendication 2 dans lequel on opère en phase liquide avec un débit de F125 à purifier correspondant à une vitesse spatiale comprise entre 4 et 20 v/h/v, avec une vitesse de passage de 5 à 40 cm/min.

## Patentansprüche

1. Verfahren zur Reinigung von Chlortrifluorethylen (F1113) enthaltendem Pentafluorethan (F125), **dadurch gekennzeichnet, daß** man einen zu reinigenden Strom von F125 durch ein Bett von Aktivkohle leitet, wobei die Aktivkohle eine spezifische Oberfläche zwischen 800 und 1.500 m²/g und eine Mikroporosität in der Größenordnung von 50 bis 90 %, vorzugsweise 70 bis 85 %, hat.

2. Verfahren nach Anspruch 1, in dem bei einer Temperatur zwischen -20 und +80 °C, vorzugsweise zwischen 10 und 40 °C und bei einem Druck von 100 bis 2.200 kPa, gearbeitet wird.

3. Verfahren nach Anspruch 2, bei dem man in der Gasphase mit einer Zufuhr des zu reinigenden F125, entsprechend einer Raumgeschwindigkeit zwischen 50 und 1.500 v/h/v mit einer Durchschlußgeschwindigkeit von 10 bis 2.500 cm/min, arbeitet.

4. Verfahren nach Anspruch 2, bei dem man in flüssiger Phase mit einem Zufluß des zu reinigenden F125, entsprechend einer Raumgeschwindigkeit zwischen 4 und 20 v/h/v mit einer Durchschlußgeschwindigkeit von 5 bis 40 cm/min, arbeitet.

## Claims

1. Process for the purification of a pentafluoroethane (F125) containing chlorotrifluoroethylene (F1113), **characterized in that** a stream of the F125 to be purified is passed through a bed of active carbon, the said active carbon having a specific surface of between 800 and 1500 m²/g and a microporosity of the order of 50 to 90 %, preferably 70 to 85 %.

2. Process according to Claim 1, in which the operation is carried out at a temperature of between -20 and +80°C, preferably between 10 and 40°C and at a pressure ranging from 100 to 2200 kPa.

3. Process according to Claim 2, in which the operation is carried out in gas phase with a flow rate of F125 to be purified corresponding to a space velocity of between 50 and 1500 v/h/v, with a flow velocity of 10 to 2500 cm/min.

4. Process according to Claim 2, in which the operation is carried out in liquid phase with a flow rate of F125 to be purified corresponding to a space velocity of between 4 and 20 v/h/v, with a flow velocity of 5 to 40 cm/min.
